Europäisches Patentamt

European Patent Office

Office européen des brevets

(11) Publication number: **0 508 282 A2**

# EUROPEAN PATENT APPLICATION

(21) Application number: **92105559.6**

(22) Date of filing: **31.03.92**

(51) Int. Cl.5: **C07K 15/28**, C12N 5/18,
A61K 39/00, G01N 33/574

(30) Priority: **01.04.91 JP 68378/91**

(43) Date of publication of application:
**14.10.92 Bulletin 92/42**

(84) Designated Contracting States:
**DE FR GB IT**

(71) Applicant: **KYOWA HAKKO KOGYO CO., LTD.**
**6-1, Ohtemachi 1-chome**
**Chiyoda-ku Tokyo-to(JP)**

(72) Inventor: **Hanai, Nobuo**
**3-3-3-202, Fujimi**
**Sagamihara-shi, Kanagawa(JP)**
Inventor: **Furuya, Akiko**
**1210 i15-405; Kisomachi**
**Machida-shi, Tokyo(JP)**

(74) Representative: **Kinzebach, Werner, Dr. et al**
**Patentanwälte Reitstötter, Kinzebach und**
**Partner Sternwartstrasse 4 Postfach 86 06 49**
**W-8000 München 86(DE)**

(54) **Anti-idiotypic monoclonal antibodies.**

(57) Anti-idiotypic monoclonal antibodies are provided which react specifically with anti-sialyl-Lewis A monoclonal antibodies and are useful in cancer diagnosis and cancer therapy.

EP 0 508 282 A2

## FIELD OF THE INVENTION

This invention relates to anti-idiotypic monoclonal antibodies which specifically bind to anti-sialyl-Lewis A monoclonal antibodies, which are useful in cancer diagnosis and treatment.

## BACKGROUND OF THE INVENTION

The sialyl Lewis A (hereinafter, sialyl-Le$^a$) antigen is an antigen of the carbohydrate type which is produced in a variety of cancer cells, expressed on the cell membrane of such cells and extracellularly excreted, and is detected in the sera of cancer patients [(Cancer Research, 43, 5489-5492 (1983)].

Among monoclonal antibodies against the sialyl-Le$^a$ antigen which are known, there are NS19-9 [J. Biol. Chem., 257, 14365-14369 (1982)], CA50 [British Medical Journal, 288, 1479-1482 (1984)] and AMC-462 (hereinafter, KM231) [Anticancer Research, 8, 329-334 (1988); JP-A-63-21562 corresponding to U.S. Patent 5,051,355 (the term "JP-A" used herein means an unexamined published Japanese patent application)]. Furthermore, it is considered that such monoclonal antibodies as MSW113 [J. Biochem., 104, 817-821 (1988)], CC3C195 [Arch. Biochem. Biophys., 255, 214-216 (1987)] and Span-1 [Cancer, 60, 1636-1643 (1987)] can recognize the sialyl-Le$^a$ antigen.

These monoclonal antibodies are thought to be applicable in the serodiagnosis of cancer which comprises determining the level of sialyl-Le$^a$ antigen in the sera of cancer patients [Cancer Research, 43, 5489-5492 (1983); Anticancer Research, 9, 999-1004 (1989)] and in the treatment of cancer (JP-A-3-130235).

Anti-idiotypic antibodies recognize a variable region in an antibody obtained against a certain antigen epitope, and the variable region of an anti-idiotypic antibody is thought to be very close in three-dimensional structure to the antigen epitope. Such anti-idiotypic antibodies can be used not only in immunoassay [Science, 215, 1637-1639 (1982)] but also as vaccines for cancer therapy (PROGRESS IN VACCINOLOGY, 3, Anti-Idiotypic Vaccines).

Therefore, anti-idiotypic antibodies to an anti-sialyl-Le$^a$ monoclonal antibody, if obtained, would be useful in the treatment and/or diagnosis of cancer. However, it is very difficult to produce anti-idiotypic antibodies specifically reacting with an antibody to such a carbohydrate chain antigen as the sialyl-Le$^a$ antigen. An anti-idiotypic monoclonal antibody to the stage-specific embryonic antigen (SSEA-1 antigen) [Proceedings of the 48th Annual Meeting of the Japanese Cancer Association, Abstract No. 1662, 298 (1989)] is the only one that has been reported.

No anti-idiotypic antibody, whether polyclonal or monoclonal, specifically reacting with an antibody to the sialyl-Le$^a$ antigen is known.

Accordingly it is an object of the invention to provide anti-idiotypic monoclonal antibodies to an anti-sialyl-Le$^a$ monoclonal antibody which are useful in the diagnosis and/or treatment of cancer.

## SUMMARY OF THE INVENTION

The invention provides anti-idiotypic monoclonal antibodies specifically reacting with an anti-sialyl-Le$^a$ monoclonal antibody.

The invention also provides a method for identifying a patient with cancer comprising the steps of: (a) contacting a sample to an anti-idiotypic monoclonal antibody which specifically binds to an anti-sialyl-Lewis A monoclonal antibody; and (b) detecting the formation of an anti-idiotypic monoclonal antibody/anti-sialyl-Lewis A antibody complex.

Typically, a first anti-idiotypic monoclonal antibody is immobilized on a solid phase. A sample is then contacted to said solid phase, and then a second anti-idiotypic monoclonal antibody which is labeled is contacted to said solid phase. The formation of an anti-sialyl-Lewis A antibody/labeled anti-idiotypic monoclonal antibody complex is detected by means of the label.

In another embodiment, the invention, after the sample is contacted to the solid phase, a labeled anti-human immunoglobulin specific antibody is contacted to the solid phase. The formation of an anti-sialyl-Lewis A antibody/labeled anti-human immunoglobulin specific antibody complex is detected by means of the label.

The invention also provides a kit comprising (a) an anti-idiotypic monoclonal antibody specifically binding anti-sialyl-Lewis A monoclonal antibody on a solid phase; and (b) a labeled anti-human immunoglobulin specific antibody, or in another embodiment, a labeled anti-idiotypic monoclonal antibody specifically binding anti-sialyl-Lewis A monoclonal antibody.

In another embodiment, the invention provides a method of treating cancer comprising administering to a patient an effective amount of an anti-idiotypic monoclonal antibody specifically binding to an anti-sialyl-Lewis A antibody. Advantageously, the anti-idiotypic monoclonal antibody is KM468.

## BRIEF DESCRIPTION OF THE DRAWINGS

Fig. 1 shows the rates of inhibition of the binding reaction between the sialyl-Le$^a$ antigen and KM231 by the hybridoma clones 1-4 (KM467), 5-12, 7-14, 8-12, 15-11, 20-13 (KM468), 28-16 (KM469) and 76-5 (KM470). B1 stands for a blank, and NMS for untreated mouse-derived serum.

The values $10^{-2}$, $10^{-3}$ and $10^{-4}$ given for the antiserum mean the dilution factors 100, 1,000 and 10,000, respectively, for the immunized mouse serum.

Fig. 2 shows the reactions of some anti-idiotypic monoclonal antibodies with various monoclonal antibodies.

The number 1 stands for KM305, 2 for KM230, 3 for KM231, 4 for KM232, 5 for KM235, 6 for KM237, 7 for KM360, 8 for KM191, 9 for KM201, 10 for KM206, 11 for KM225, 12 for NS19-9, and P3-U1 for the culture supernatant obtained from cultured p3-U1 myeloma cells.

Fig. 3 shows the rates of inhibition of the reactivity of some anti-idiotypic monoclonal antibodies for the sialyl-Le$^a$ oligosaccharide.

The symbol o stands for 1-4 (KM467), o for 7-14, △ for 8-12, ◊ for 20-13 (KM468), ♦ for 28-16 (KM469) and □ for 76-5 (KM470).

Fig. 4 shows the effect of an anti-idiotypic monoclonal antibody as a vaccine (effect 1; induction of an anti-GM$_2$ antibody).

Fig. 4(a) shows the reactivity of antisera (100-fold diluted) obtained by immunizing 8 rats with the KM468-Hemocyamine Keyhole Linpet conjugate for the sialyl-Le$^a$ glycolipid. KM231 (10 $\mu$g/ml) was used as a positive control, and a 100-fold dilution of untreated rat-derived serum (NRS) as a negative control.

Fig. 4(b) shows the inhibitory effects of the antisera obtained on the binding reaction between KM468 and KM231. A 100-fold dilution of untreated rat-derived serum (NRS) was used as a negative control.

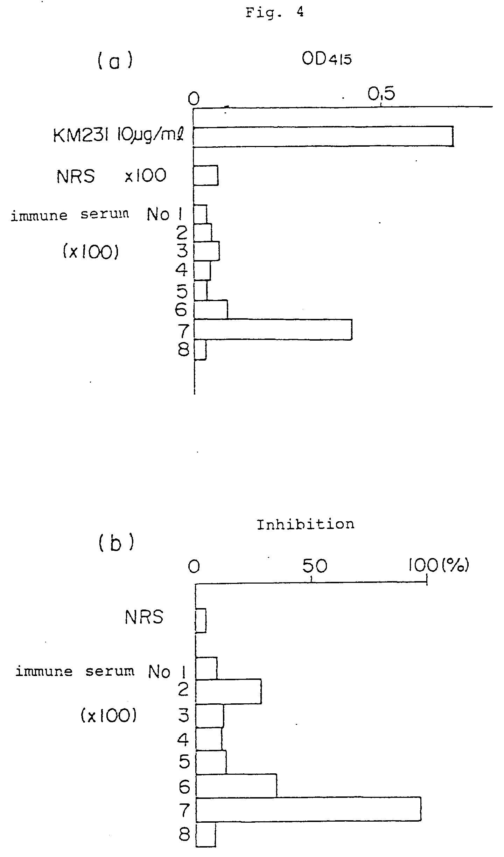

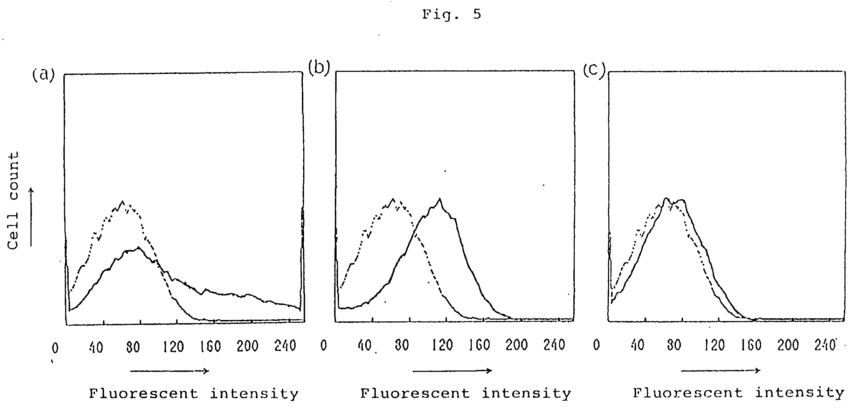

Fig. 5(b) shows the results (solid line) of an experiment where a 20-fold dilution of the No. 7 rat-derived serum, which gave the highest antibody titer among the 8 rats immunized with the KM468-KLH conjugate (cf. Fig. 4), was reacted with KATO-3 cells (human gastric cancer cells) and the reaction was analyzed by flow cytometry. Fig. 5(a) shows the reaction pattern (solid line) for KM231 (10 $\mu$g/ml) used as a positive control, and Fig. 5(c) shows the reaction pattern (solid line) for untreated rat-derived serum (20-fold dilution) used as a negative control. In the figures (a), (b) and (c), each dotted line shows the pattern of reaction of KATO-3 cells in the absence of the antibody.

Fig. 6 shows the life span-prolonging effect of KM468 against mouse lymphoma cells.

Fig. 7 shows the anti-sialyl-Le$^a$ monoclonal antibody titers of sera from gastric cancer patients and normal human sera. The abbreviation hIg is for human immunoglobulin used as a control.

DETAILED DESCRIPTION OF THE INVENTION

The subject invention provides anti-idiotypic monoclonal antibodies which are useful in cancer diagnosis and cancer therapy.

Hybridomas are produced by fusion of splenocytes of an animal immunized with an anti-sialyl-Le$^a$ monoclonal antibody-carrier protein conjugate with mouse myeloma cells, and a hybridoma is selected which inhibits the reaction of the sialyl-Le$^a$ antigen and the anti-sialyl-Le$^a$ monoclonal antibody. The hybridoma is cultured in a medium or administered to an animal for the formation of ascites tumor to collect monoclonal antibodies from the culture broth or ascitic fluid. Among the monoclonal antibodies obtained, one which reacts with the anti-sialyl-Le$^a$ monoclonal antibody but does not react with monoclonal antibodies to other antigens and whose binding to the anti-sialyl-Le$^a$ monoclonal antibody is inhibited by an oligosaccharide which is the epitope of the sialyl-Le$^a$ antigen is selected as an anti-idiotypic monoclonal antibody.

The anti-idiotypic monoclonal antibodies thus obtained can serve as vaccines for effectively preventing the proliferation of tumor cells and further can be used in cancer diagnosis in the manner of sandwich assay or inhibition assay.

In the following, a method of producing the anti-idiotypic monoclonal antibodies of this invention is described in further detail.

(1) Immunization of animals and preparation of antibody-producing cells

Mice or rats of 3-20 weeks of age are immunized with the antigen [a conjugate between an anti-sialyl-Le$^a$ monoclonal antibody and a carrier protein such as Hemocyanine Keyhole Linpet (hereinafter, KLH; Calbiochem), bovine serum albumin, thyroglobulin, ovalbumin or human serum albumin], and antibody-producing cells are collected from the spleen, lymph node or peripheral blood of the animals.

The immunization is performed generally by administering the antigen together with an appropriate adjuvant, such as complete Freund's adjuvant, or aluminum hydroxide gel plus pertussis vaccine, to the animals subcutaneously, intravenously or intraperitoneally.

Following the first antigen administration, the antigen is administered repeatedly 5 to 10 times at one- to two-week intervals. Three to seven days after each administration, a blood sample is taken from each animal from the venous plexus of the fundus of the eye, and the serum derived from the sample blood is tested, for example, by enzyme immunoassay [cf. Enzyme-linked Immunosorbent Assay (ELISA)", published by Igaku Shoin, Tokyo 1976)] as to whether it is reactive with the antigen. A mouse or rat whose serum shows a sufficient antibody titer against the antigen used for immunization is submitted for use as a splenocyte source.

For submission to fusion between splenocytes and myeloma cells, the spleen of the immunized mouse or rat is excised 3 to 7 days after the final administration of the antigenic substance and splenocytes of the spleen are collected. Thus, the spleen is cut to pieces in MEM (Nissui Pharmaceutical), and cells are unbound using a forceps. After centrifugation (1,200 rpm, 5 minutes), the supernatant is discarded, and the sediment is treated with Tris-ammonium chloride buffer (pH 7.65) for 1 to 2 minutes for eliminating erythrocytes. The remaining cells are washed three times with MEM and submitted as splenocytes for cell fusion.

(2) Preparation of myeloma cells

Mouse-derived established cell lines are used as the myeloma cells. Thus, for instance, the 8-azaguanine-resistant mouse (BALB/c-derived) myeloma cell lines P3-X63Ag8-U1 (P3-U1) [Current Topics in Microbiology and Immunology-1; European Journal of Immunology 6, 511-519 (1976)], SP2/O-Ag14 (SP-2) [Nature, 276, 269-270 (1978)], P3-X63-Ag8653 (653) [Journal of Immunology 123, 1548-1550 (1979)] and P3-X63-Ag8 (X63) [Nature, 256, 495-497 (1975)] may be used. These cell lines are subcultured in an 8-azaguanine medium [prepared by supplementing RPMI-1640 medium with glutamine (1.5 mM), 2-mercaptoethanol (5 x $10^{-5}$ M), gentamicin (10 $\mu$g/ml) and fetal calf serum (FCS) (CSL; 10%) and further supplementing the resulting normal medium (hereinafter "the normal medium") with 8-azaguanine (15 $\mu$g/ml)]. Three to four days before cell fusion, subculture is performed in the normal medium to thereby ensure a cell number of not less than 2 x $10^7$ cells on the day of cell fusion.

(3) Cell fusion

The antibody-producing cells obtained as described under (1) and the myeloma cells obtained as described under (2) are respectively washed well with MEM or PBS (1.83 g of disodium phosphate, 0.21 g of monopotassium phosphate and 7.65 g of sodium chloride per liter of distilled water, pH 7.2), and mixed in a proportion of 5 to 10 antibody-producing cells per myeloma cell, and the mixture is subjected to centrifugation (1,200 rpm, 5 minutes). The supernatant is discarded, the sediment (cells) is thoroughly disintegrated, a mixture of 2 g of polyethylene glycol-1,000 (PEG-1,000), 2 ml of MEM and 0.7 ml of dimethyl sulfoxide is added to the cells with stirring in an amount of 0.2 to 1 ml/$10^8$ antibody-producing cells at 37°C, then several 1 to 2 ml of MEM are added at 1- to 2-minute intervals, and the whole amount is made 50 ml by further addition of MEM. After centrifugation (900 rpm, 5 minutes), the supernatant is discarded, the cells are loosened gently and then suspended in 100 ml of HAT medium [prepared by supplementing the normal medium with hypoxanthine ($10^{-4}$ M), thymidine (1.5 x $10^{-5}$ M) and aminopterine (4 x $10^{-7}$ M)] by repeated drawing up into and discharging from a graduated pipette.

This suspension is distributed in 100-$\mu$l portions into each well of 96-well incubation plates, and incubation is carried out in a 5% $CO_2$ incubator at 37°C for 7 to 14 days.

After incubation, a portion of the culture supernatant is taken from each well and subjected to enzyme immunoassay, for instance. Wells giving a supernatant capable of inhibiting the binding reaction between the sialyl-Le[a] antigen and the anti-sialyl-Le[a] monoclonal antibody used for immunization are selected and subjected to cloning, which is repeated twice by the limiting dilution method [using HT medium (HAT medium minus aminopterine) for the first cloning and the normal medium for the second]. Strains for which a high antibody titer is constantly observed are selected as anti-idiotypic monoclonal antibody-producing hybridoma strains.

(4) Preparation of monoclonal antibodies

The anti-idiotypic monoclonal antibody-producing hybridoma cells obtained as described under (3) are intraperitoneally injected into 8 to 10-week-old mice or nude mice treated with pristane [by intraperitoneal administration of 0.5 ml of 2,6,10,14-tetramethylpentadecane (pristane) followed by 2 weeks of feeding] at a dose of 2 x $10^6$ to 5 x $10^7$ cells per animal. The hybridoma causes ascites tumor in 10 to 21 days. The ascitic fluid is collected from the mice, centrifuged (3,000 rpm, 5 minutes) to remove solid matter and, after salting out with 40-50% ammonium sulfate, passed through a DEAE-Sepharose column, protein A column or Cellulofine GSL 2000 (Seikagaku Corp.) column. An IgG or IgM fraction are recovered to give a purified monoclonal antibody.

The subclass of the antibody can be determined using a mouse monoclonal antibody typing kit (Zymed Laboratories) or a rat monoclonal antibody typing kit (Nordic Immunology). The amount of protein can be determined by the Lowry method, followed by calculation based on the optical density at 280 nm.

(5) Selection of anti-idiotypic monoclonal antibodies

Purified anti-sialyl-Le[a] monoclonal antibodies and monoclonal antibodies against other antigens, each in the form of a 1 to 50 μg/ml solution, are distributed in 10 to 100-μl portions into wells of 96-well plates. The plates are kept at 4°C for 10 hours for coating thereof. Blocking is then effected using a bovine serum albumin (BSA) solution or the like. The plates are washed with PBS or PBS supplemented with 0.05% Tween-20 (hereinafter PBS-Tween), then the purified antibodies obtained as described under (4) labeled with biotin, an enzyme, an isotope or the like are distributed in 50 to 100 μl portions into the wells, and the plates are kept at room temperature for 2 hours or at 4°C for 10 hours. Thereafter a detection procedure suitable for the label is performed and that (or those) reacting only with the anti-sialyl-Le[a] monoclonal antibodies out of the antibodies used for plate coating is selected as an anti-idiotypic monoclonal antibody.

As a specific example of the anti-idiotypic monoclonal antibody as selected in the above manner, there may be mentioned the anti-idiotypic monoclonal antibody KM468 produced by the hybridoma cell line KM468. The hybridoma cell line KM468 has been deposited with the Fermentation Research Institute, Agency of Industrial Science and Technology, Japan of 1-3, Higashi 1-chome, Tsukuba-shi, Ibaraki, Japan since March 28, 1991 under the Budapest Treaty (deposit number: FERM BP-3334).

(6) Confirmation of the specificity of the anti-idiotypic monoclonal antibodies

The anti-idiotypic monoclonal antibody selected as described under (5), as a 1 to 50 μg/ml solution, is distributed in 10 to 100-μl portions into wells of a 96-well plate. The plate is kept at 4°C for 10 hours for coating thereof. Blocking is then effected using a BSA solution or the like. A sialyl-Le[a] oligosaccharide solution diluted to a concentration of 0.001 to 10 μg/ml and a 0.001 to 10 μg/ml solution of an anti-sialyl-Le[a] monoclonal antibody labeled with biotin, an enzyme, an isotope or the like are added, each in 50 to 100-μl portions, to the wells. The plate is kept at room temperature for 2 hours or at 4°C for 10 hours. Then, the plate is washed well with PBS or PBS-Tween, and a detection procedure suitable for the label is performed for judging as to whether the binding reaction between the anti-idiotypic monoclonal antibody and the anti-sialyl-Le[a] monoclonal antibody is suppressed by the sialyl-Le[a] oligosaccharide.

(7) Effect of the anti-idiotypic monoclonal antibodies as vaccines -1-

Animals are immunized with 10 to 1,000 μg (per animal) of the purified anti-idiotypic monoclonal antibody or a conjugate thereof with an appropriate carrier protein, together with an appropriate adjuvant such as complete Freund's adjuvant. After several immunizations, blood samples are collected and the sera therefrom are assayed for antibody titer for the reaction with the sialyl-Le[a] antigen. Thus, a 96-well plate is coated with the purified sialyl-Le[a] antigen, followed by blocking with a BSA solution or the like. An appropriate dilution of each immunized animal-derived serum is distributed in 50 to 100-μl portions into the wells, and the plate is kept at room temperature for 2 hours or at 4°C for 10 hours. After thorough washing of the plate, a 1 to 50 μg/ml solution of an anti-mouse or anti-rat immunoglobulin antibody raised against the immunized animal-derived immunoglobulin and labeled with biotin, an enzyme, a chemiluminescent substance, a radioactive compound or the like is distributed, as a second antibody and in 50 to 100-μl portions, into the wells. The plate is kept at room temperature for 2 hours or at 4°C for 10 hours. Then, after thorough washing of the plate, a reaction suitable for the label on the second antibody is carried out and the anti-sialyl-Le[a] antibody titer in the serum is determined.

(8) Effect of the anti-idiotypic monoclonal antibodies as vaccines -2-

Untreated animals or animals carrying a transplanted syngeneic tumor expressing the sialyl-Le[a] antigen were immunized several times with 10 to 1,000 μg (per animal) of the purified anti-idiotypic monoclonal antibody or a conjugate thereof with an appropriate carrier protein, in each case together with an appropriate adjuvant such as complete Freund's adjuvant. When untreated animals are immunized in that manner, the syngenic tumor expressing the sialyl-Le[a] antigen is transplanted into the animals at this time point. The effect of the anti-idiotypic monoclonal antibody as an antitumor vaccine is evaluated by comparing the anti-idiotypic monoclonal antibody-immunized animal group and a control monoclonal antibody-immunized animal group with respect to the growth of tumor or death of animals.

(9) Application of the anti-idiotypic monoclonal antibodies in serodiagnosis

a. Sandwich assay

A 1 to 50 μg/ml solution of the purified anti-idiotypic monoclonal antibody is distributed in 10 to 100 μl portions into wells of a 96-well plate, and the plate is kept at 4°C for 10 hours for coating thereof with said antibody (first antibody). Then, blocking is effected with a BSA solution or the like. An appropriate dilution of human serum or a standard solution is added to the wells (50 to 100 $\mu l$ per well), and the plate is kept at room temperature for 2 hours or at 4°C for 10 hours and then washed well with PBS or PBS-Tween. A 1 to 50 $\mu g/ml$ solution of the anti-idiotypic monoclonal antibody, either unlabeled or labeled with biotin, an enzyme, a chemiluminescent substance, a radioactive compound or the like, is distributed in 50 to 100 $\mu l$ portions into the wells (second antibody). The plate is kept at room temperature for 2 hours or at 4°C for 10 hours and then washed well. A reaction suitable for the label on the second antibody is then carried out, and the amount of anti-sialyl-Le$^a$ antibody in the human serum is calculated based on the concentration of the anti-sialyl-Le$^a$ monoclonal antibody in the standard solution.

b. Inhibition assay

A 1 to 50 $\mu g/ml$ solution of the purified anti-idiotypic monoclonal antibody is distributed in 10 to 100 $\mu l$ portions into wells of a 96-well plate, and the plate is kept at 4°C for 10 hours for coating thereof with said antibody. Then, blocking is effected with a BSA solution or the like. An appropriate dilution of human serum and a solution of the anti-sialyl-Le$^a$ monoclonal antibody labeled with an enzyme, a chemiluminescent substance, a radioactive compound or the like (0.01 to 10 $\mu g/\mu l$) are each distributed in 50 to 100 $\mu l$ portions into wells. The plate is kept at room temperature for 2 hours or at 4°C for 10 hours and then washed well. A reaction suitable for label on the anti-sialyl-Le$^a$ monoclonal antibody is then carried out and the amount of anti-sialyl-Le$^a$ antigen in the human serum is determined based on inhibition of the reaction.

The anti-idiotypic monoclonal antibody can be used for treatment of cancer such as gastric cancer, large intestine cancer, pancreatic cancer, breast cancer, lung adenocarcinoma and the like. The antibody is subcutaneously administered to a patient in a dose of 0.5 to 4 mg once a week for 5 weeks.

The following examples are further illustrative of the invention, but are not construed to limit the scope of the invention.

Example 1

(1) Immunization of animals and preparation of antibody-producing cells

The anti-sialyl-Le$^a$ monoclonal antibody KM231 and KLH (carrier protein) were mixed in a protein ratio of 1:5 and treated with 0.02 M glutaraldehyde in the presence of 0.1 M ammonium acetate according to a known method [Proc. Soc. Exp. Biol. Med., 148, 784-789 (1975)] to give a conjugate (KM231-KLH conjugate), which was dialyzed against PBS and then used as an antigen. Separately, m-maleimidebenzoyl-N-hydroxysuccil (MBS; Nakalai Tesque) was reacted with KLH and the reaction product was purified on a Sephadex G25 column. The purified product was mixed with the monoclonal antibody KM231 in a ratio of 5:1 and the mixture was subjected to conjugate formation by a known method [Cell, 28, 477-487 (1982)]. The conjugate was dialyzed against PBS and used as an antigen. (Either of the methods can give an antigen equally applicable in the subsequent procedure.) $F_1$ mice (C57BL/b male x BALB/c female) were given 100 $\mu g$ of the KM231-KLH conjugate (hereinafter, KM231-KLH) together with 2 mg of an aluminum gel and 1 x 10$^9$ cells of pertussis vaccine (Chiba Serum Institute) (per animal). After 2 weeks, weekly administration of 100 $\mu g$ of KM231-KLH was started. After 6 administrations in all, blood was sampled from the venous plexus of the fundus of the eye and tested for serum antibody titer by enzyme immunoassay. Three days after the final immunization, the spleen was excised from a mouse showing a sufficient antibody titer.

The spleen was cut to pieces in MEM (Nissui Pharmaceutical) and cells were unbound using a forceps. After centrifugation (1,200 rpm, 5 minutes), the supernatant was discarded, and the sediment was treated with Tris-ammonium chloride buffer (pH 7.65) for 1 to 2 minutes for eliminating erythrocytes. The cells were then washed three times with MEM and submitted for cell fusion.

Enzyme immunoassay

A 1 to 50 $\mu g/ml$ solution of the antigen prepared as described in Example 1-(1) was distributed in 10 to 100 $\mu l$ portions into wells of a 96-well plate for EIA (Greiner), and the plate was kept at 4°C for 10 hours for coating. Then, a PBS solution containing 1% BSA (BSA-PBS) was distributed in 100 to 200 $\mu l$ portions into the wells. The plate was kept at room temperature for 1 to 2 hours or at 4°C for 10 to 30 hours for blocking the protein-binding residues remaining on the plate. The BSA-PBS was then discarded, the plate was washed well with PBS, and samples (mouse sera, hybridoma culture supernatants, purified monoclonal antibodies) diluted with BSA-PBS were respectively distributed in 20 to 100 $\mu l$ portions into the wells (each as a first antibody). The plate was kept at room temperature for 2 to 3 hours and then washed well with PBS or PBS-Tween. A peroxidase-labeled anti-mouse immunoglobulin antibody (DAKO) or peroxidase-labeled anti-rat immunoglobulin antibody (DAKO), as a second antibody, was distributed in 50 to 100 $\mu l$ portions into

the wells, and the plate was kept at room temperature for 2 hours.

After thorough washing of the plate with PBS, color development was caused using an ABTS substrate solution [prepared by dissolving 550 mg of 2,2'-azinobis(3-ethylbenzothiazoline-6-sulfonic acid) diammonium salt in 1 liter of 0.1 M citrate buffer (pH 4.2) and adding, just prior to use, hydrogen peroxide to the solution to a concentration of 1 $\mu$l/ml], and optical density ($OD_{415\ nm}$) measurement was performed.

(2) Preparation of mouse myeloma cells

The 8-azaguanine-resistant mouse myeloma cell line P3-U1 was cultured in the normal medium and not less than $2 \times 10^7$ cells were thereby secured at the time of cell fusion and submitted for cell fusion as a parent strain.

(3) Hybridoma formation

The mouse splenocytes obtained as described in Example 1-(1) and the myeloma cells obtained as described in Example 1-(2) were mixed in a ratio of 10:1, and the mixture was centrifuged (1,200 rpm, 5 minutes). The supernatant was discarded, and the cells (sediment) were thoroughly loosened. A mixed solution composed of 2 g of polyethylene glycol-1,000 (PEG-1,000), 2 ml of MEM and 0.7 ml of dimethyl sulfoxide was added to the cells with stirring at 37°C in an amount of 0.2 to 1 ml per $10^8$ mouse splenocytes, followed by addition of several 1 to 2-ml portions of MEM at 1 to 2-minute intervals. Thereafter, the total volume was made 50 ml by addition of MEM. After centrifugation (900 rpm, 5 minutes), the supernatant was discarded, and the cells were gently loosened and then gently suspended in 100 ml of HAT medium by repeated drawing up into and discharging from a graduated pipette.

The suspension was distributed in 100-$\mu$l portions into wells of a 96-well incubation plate and incubation was performed in a 5% $CO_2$ incubator (under 5% $CO_2$) at 37°C for 10 to 14 days. Each culture supernatant was examined by the hybridoma selection method mentioned below. Activity-positive wells were selected and, for them, cloning was repeated twice using HT medium and the normal medium, respectively. Based on the results of enzyme immunoassay, hybridomas producing a monoclonal antibody capable of inhibiting the binding reaction between KM231 and the anti-sialyl-Le$^a$ antigen were selected.

Hybridoma selection

A 1 to 50 $\mu$g/ml solution of an antigen purified from the ascitic fluid of a human gastric cancer patient by gel chromatography (GCL-2000, Seikagaku Corp.) was distributed in 10 to 100 $\mu$l portions into wells of a 96-well EIA plate (Greiner), and the plate was kept at 4°C for 10 hours for plate coating.

Then, 1% BSA-PBS was distributed in 100 to 200 $\mu$l portions into the wells, and the plate was kept at room temperature for 1 to 2 hours for blocking the protein-binding residues remaining on the plate. Thereafter, BSA-PBS was discarded, the plate was washed well with PBS and then the hybridoma culture supernatant (first antibody) was distributed in 20 to 100-$\mu$l portions into the wells. The plate was kept at room temperature for 2 to 3 hours.

The plate was washed well with PBS or PBS-Tween, a 10 $\mu$g/ml solution of biotin-labeled KM231 (second anti-body) was distributed in 50 to 100-$\mu$l portions into the wells, and the plate was kept at room temperature for 2 hours.

After thorough washing with PBS, the plate was treated with an avidin-peroxidase conjugate (Vector Laboratories Inc.) (50 to 100 $\mu$l/well) at room temperature for 1 to 2 hours. After thorough washing of the plate with PBS, color development was caused using the ABTS substrate solution, which was followed by optical density ($OD_{415\ nm}$) measurement.

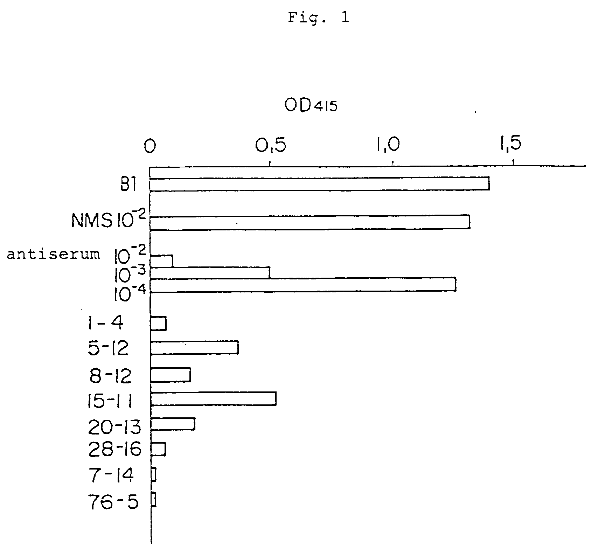

As shown in Fig. 1, eight monoclonal antibodies capable of inhibiting the binding of KM231 to the sialyl-Le$^a$ antigen were selected, namely the monoclonal antibodies 1-4 (KM467, IgG$_1$), 5-12 (IgG$_1$), 7-14 (IgG$_1$), 8-12 (IgG$_1$), 15-11 (IgM), 20-13 (KM468, IgG$_1$), 28-16 (KM469, IgG$_1$) and 76-5 (KM470, IgG$_1$).

(4) Purification of monoclonal antibodies

The hybridoma strains obtained as described under (3) were respectively administered to pristane-treated female nude mice of 8 weeks of age by intraperitoneal injection at a dose of 5 to 10 $\times 10^6$ cells per animal. The hybridomas caused ascites tumor formation in 10 to 21 days. The ascitic fluid was collected from each ascitic fluid-carrying mouse (1 to 8 ml per animal) and centrifuged (3,000 rpm, 5 minutes) for removing solid matter. In the case of a monoclonal antibody of the class IgM, the supernatant was subjected to salting out with 50% ammonium sulfate, then dialyzed against PBS supplemented with 0.5 M sodium chloride and passed through a Cellulofine GSL2000 (Seikagaku Corp.) column (bed volume 750 ml) at a flow rate of 15 ml per hour, and an IgM fraction was collected to give a purified monoclonal antibody.

In the case of a monoclonal antibody of the class IgG, the supernatant was subjected to two repetitions of salting out effected with 50% ammonium sulfate and 40% ammonium sulfate, respectively, then dialyzed against 0.04 M phosphate buffer (pH 8.0) containing 0.03 M NaCl and applied to a DEAE Sepharose (Whatman DE52) column, followed by elution. An IgG fraction was collected to give a purified monoclonal antibody.

(5) Selection of anti-idiotypic monoclonal antibodies

A 10 $\mu$g/ml solution of the purified sample of each of the eight monoclonal antibodies selected as described under (4) was distributed in 50-$\mu$l portions into wells of a 96-well plate, and the plate was kept at 4°C for 10 hours for coating. Then, blocking was effected using 1% BSA-PBS, a biotinylated mouse monoclonal antibody solution (10 $\mu$g/ml) was distributed in 50-$\mu$l portions into the wells, and the plate was kept at room temperature for 2 hours. The biotinylated mouse monoclonal antibodies used were the anti-sialyl-Le$^a$ monoclonal antibodies KM230 (IgG$_1$), KM231 (IgG$_1$), KM191 (IgM) and KM206 (IgM) [Anticancer Research, 8,-329-334 (1988)] and NS19-9 (Fuji Rebio) as well as the following mouse monoclonal antibodies obtained by immunization with human gastric cancer tissue membrane components and recognizing other antigens than the sialyl-Le$^a$ antigen: KM201 (IgM), KM225 (IgG$_1$), KM232 (IgG$_{2b}$), KM235 (IgG$_1$), KM237 (IgG$_1$), KM305 (IgG$_1$) and KM360 (IgM).

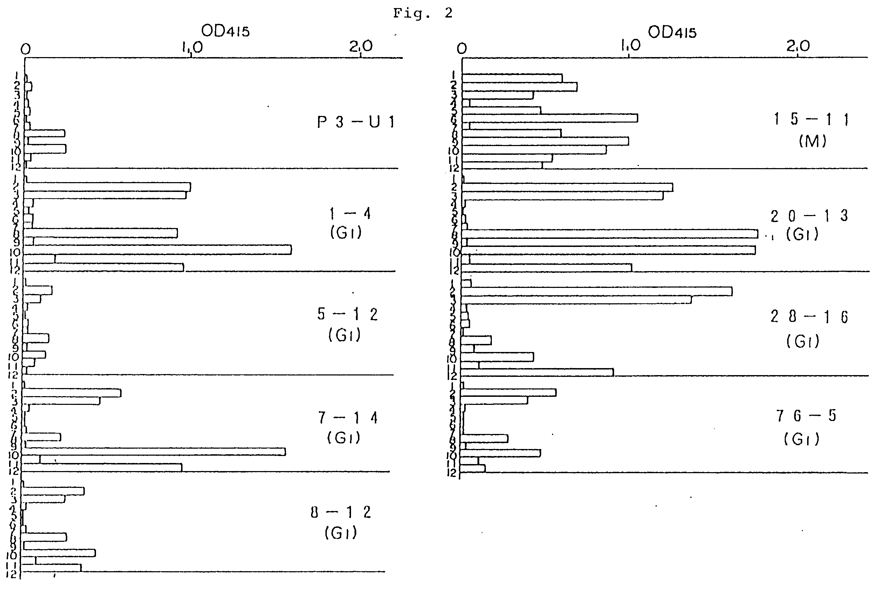

After thorough washing with PBS, the plate was subjected to reaction with an avidin-peroxidase conjugate (Vector Laboratories) (50 $\mu$l per well). The reaction was carried out at room temperature for 1 hour. After thorough washing with PBS, color development was caused using the ABTS substrate solution, as in enzyme immunoassay, and optical density (OD$_{415\ nm}$) measurement was performed. As shown in Fig. 2, the four monoclonal antibodies KM467, KM468, KM469 and KM470 reacted only with the five anti-sialyl-Le$^a$ monoclonal antibodies.

(6) Specificity testing of the anti-idiotypic monoclonal antibodies

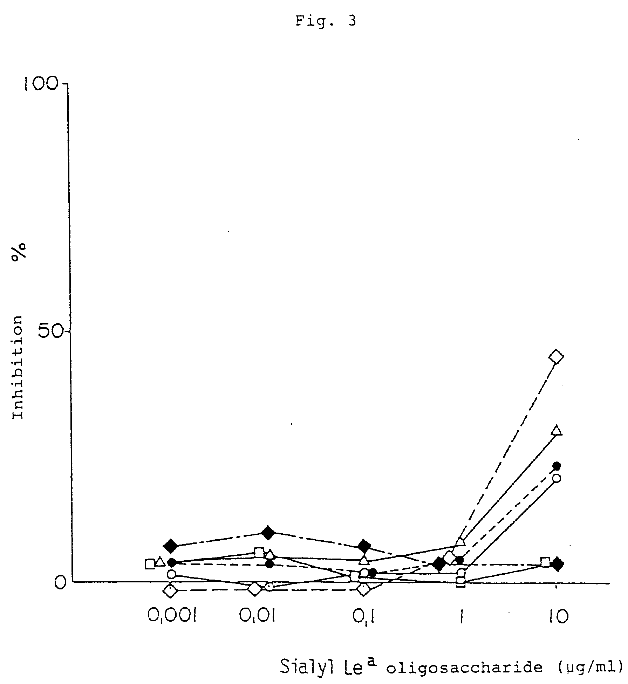

A 10 $\mu$g/ml solution of the purified sample of each of the eight monoclonal antibodies selected as described under (3) was distributed in 50-$\mu$l portions into wells of a 96-well plate, and the plate was kept at 4°C for 10 hours for coating. Then, blocking was effected using 1% BSA-PBS, 50 $\mu$l of a 0.05 $\mu$g/ml solution of biotinylated KM231 and 50 $\mu$l of a 0.001 to 10 $\mu$g/ml solution of a sialyl-Le$^a$ oligosaccharide (Biocarv Chemicals) were added to each well simultaneously, and the plate was kept at 4°C for 10 hours. After thorough washing with PBS, the plate was subjected to reaction with an avidin-peroxidase conjugate (Vector Laboratories) (50 $\mu$l per well). The reaction was carried out at room temperature for 1 hour. After thorough washing with PBS, color development was caused using the ABTS substrate solution, as in enzyme immunoassay, and optical density (OD$_{415\ nm}$) measurement was performed. As shown in Fig. 3, KM468, 8-12, 7-14 and 1-4 were inhibited, in that order, from reacting with biotinylated KM231 by the sialyl-Le$^a$ oligosaccharide.

Example 2

(1) Effect of an anti-idiotypic monoclonal antibody as a vaccine - 1 -

Eight rats were immunized once a week and four times in all with 50 $\mu$g (per animal) of a KM468-KLH conjugate prepared in the same manner as the KM231-KLH conjugate described in Example 1-(1). Complete Freund's adjuvant was supplementally used only in the first immunization. Three days after immunization, blood samples were taken and the sera therefrom were assayed for the antibody titer for the reaction with a sialyl-Le$^a$ antigen, as follows. The sialyl-Le$^a$ glycolipid was purified from KATO-3 cells by the method already reported [Anticancer Research, 8, 329-334 (1988)]. The purified sialyl-Le$^a$ glycolipid (2 nmol) was dissolved in 2 ml of ethanol containing cholesterol (2.5 ng) and phosphatidylcholine (5 ng), the solution was distributed in 20-$\mu$l portions into wells of a 96-well plate, and the plate was dried using a dryer to effect coating thereof. Then, after blocking with 1% BSA-PBS, 100-fold diluted sera from the immunized rats were distributed in 50-$\mu$l portions into the wells, and the plate was kept at room temperature for 2 hours. After thorough washing, the plate was subjected to reaction with a peroxidase-labeled anti-rat immunoglobulin (DAKO) (50 $\mu$l per well). The reaction was carried out at room temperature for 2 hours. After thorough washing with PBS, color development was caused using the ABTS substrate solution, as in enzyme immunoassay, and optical density (OD$_{415\ nm}$) measurement was performed. As shown in Fig. 4 (a), a rat serum (No. 7) from one of the 8 animals was found to have a high antibody titer against the sialyl-Le$^a$ glycolipid.

A 10 $\mu$g/ml solution of KM468 was distributed in 50-$\mu$l portions into wells of a 96-well plate, and the plate was kept at 4°C for 10 hours for coating with KM468. Then, after blocking with 1% BSA-PBS, 50 $\mu$l of a 0.05 $\mu$g/ml solution of biotinylated KM231 and 50 $\mu$l of 100-fold diluted serum from each of the immunized rats were added to the wells simultaneously. The plate was kept at 4°C for 10 hours, then washed well with PBS, and subjected to reaction with an avidin-peroxidase conjugate (Vector Laboratories) (50 $\mu$l per well). The reaction was carried out at room temperature for 1 hour. After thorough washing with PBS, color development was caused using the ABTS substrate solution, as in enzyme immunoassay, and optical density (OD$_{415 \, nm}$) measurement was performed. As shown in Fig. 4 (b), the same serum (No. 7) that had been found to have a high antibody titer against the sialyl-Le$^a$ glycolipid was found to strongly inhibit the binding reaction between KM468 and KM231.

Furthermore, this No. 7 serum was 20-fold diluted and allowed to react with about $10^6$ KATO-3 human gastric cancer cells at 4°C for 30 minutes. After washing with three portions of PBS, the cells were allowed to react with a fluorescein isothiocyanate-labeled anti-rat immunoglobulin (Vector Laboratories) at 4°C for 15 minutes. After washing with three portions of PBS, the cells were subjected to analysis using a flow cytometer (JASCO). As shown in Fig. 5, the No. 7 serum reacted with KATO-3 cells.

(2) Effect of an anti-idiotypic monoclonal antibody as a vaccine - 2 -

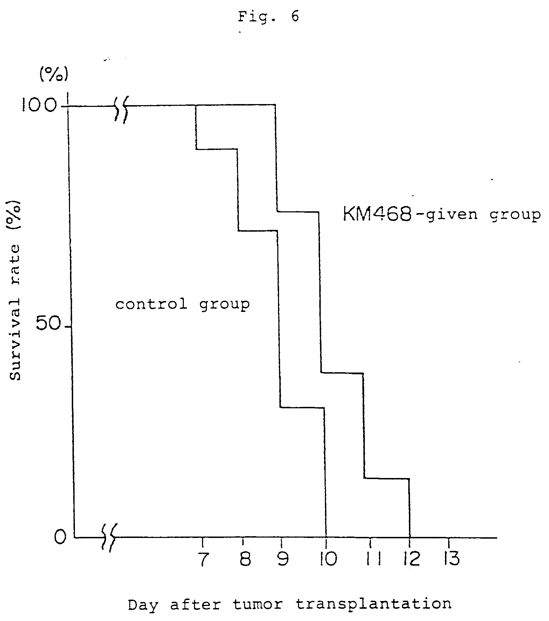

The DBA/2 mice were immunized four times with 50 $\mu$g (per animal) of the KM468-KLH conjugate. Complete Freund's adjuvant was supplementally used only in the first immunization. Three days after immunization, 5 x $10^6$ P388 mouse lymphoma cells were transplanted subcutaneously into each mouse. Control mice were immunized in the same manner with 50 $\mu$g (per animal) of a conjugate between a mouse monoclonal antibody to salmon growth hormone (JP-A-62-143693) and KLH, followed by transplantation of P388 mouse lymphoma cells. As shown in Fig. 6, a statistically significant life-prolonging effect (P<0.05) was noted in the mice immunized with KM468-KLH conjugate as compared with the control mice.

Example 3

Application of an anti-idiotypic monoclonal antibody in serodiagnosis (detection of an anti-sialyl-Le$^a$ monoclonal antibody)

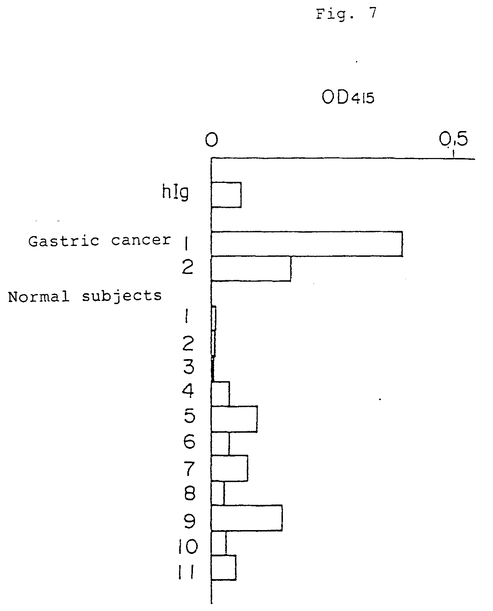

A 10 $\mu$g/ml solution of KM468 was distributed in 50-$\mu$l portions into wells of a 96-well plate, and the plate was kept at 4°C for 10 hours for plate coating with KM468 as a first antibody. Then, blocking was effected using 1% BSA-PBS. A 10-fold dilution of each human serum sample was distributed in 50-$\mu$l portions into the wells, and the plate was kept at 4°C for 10 hours. After thorough washing, a 10 $\mu$g/ml solution of biotinylated KM468 (second antibody) was distributed in 50-$\mu$l portions into the wells, and the reaction was allowed to proceed at room temperature for 2 hours. After thorough washing with PBS, the plate was subjected to reaction with an avidin-peroxidase conjugate (Vector Laboratories) (50 $\mu$l per well). The reaction was carried out at room temperature for 1 hour. After thorough washing with PBS, color development was caused using the ABTS substrate solution, as in enzyme immunoassay, and optical density (OD$_{415 \, nm}$) measurement was performed. As shown in Fig. 7, two gastric cancer patient-derived serum samples showed higher anti-sialyl-Le$^a$ antibodytiters as compared with eleven normal subject-derived serum samples.

As detailedly illustrated hereinabove, the present invention can provide anti-idiotypic monoclonal antibodies against anti-sialyl-Le$^a$ monoclonal antibodies. The anti-idiotypic monoclonal antibodies are useful in the diagnosis and treatment of cancer.

While the invention has been described in detail and with reference to specific embodiments thereof, it will be apparent to one skilled in the art that various changes and modifications can be made therein without departing from the spirit and scope thereof.

**Claims**

1. An anti-idiotypic monoclonal antibody specifically reacting with an anti-sialyl-Lewis A monoclonal antibody.

2. An anti-idiotypic monoclonal antibody as claimed in Claim 1, wherein the anti-sialyl-Lewis Amonoclonal antibody is the anti-sialyl-Lewis A monoclonal antibody KM231.

3. An anti-idiotypic monoclonal antibody as claimed in Claim 1, wherein said anti-idiotypic monoclonal antibody being a mouse monoclonal antibody belonging to the subclass IgGl.

4. An anti-idiotypic monoclonal antibody KM468 which specifically reacts with an anti-sialyl-Lewis A monoclonal antibody and which is produced by a hybridoma cell line KM468 (FERM BP-3334).

5. A hybridoma cell line KM468 (FERM BP-3334) which produces an anti-idiotypic monoclonal antibody specifically reacting with an anti-sialyl-Lewis A monoclonal antibody.

6. A tumor cell proliferation-inhibiting composition which comprises an anti-idiotypic monoclonal antibody according to one of the claims 1 to 4 specifically reacting with an anti-sialyl-Lewis A monoclonal antibody.

7. A method of immunologically assaying an anti-sialyl-Lewis A antibody which comprises using, as the antibody, an anti-idiotypic monoclonal antibody specifically reacting with an anti-sialyl-Lewis A monoclonal antibody.

8. A method of assaying an anti-sialyl-Lewis A monoclonal antibody which comprises immobilizing the mouse monoclonal antibody KM468 produced by the hybridoma cell line KM468 (FERM BP-3334) on a solid carrier; allowing an antisialyl-Lewis A monoclonal antibody to react with the immobilized mouse monoclonal antibody KM468; then allowing a labeled modification of the mouse monoclonal antibody KM468 to bind to the bound anti-sialyl-Lewis A monoclonal antibody; and quantitating the label on the bound mouse monoclonal antibody KM468.

9. A method for identifying a patient with cancer comprising the steps of:
    (a) contacting a sample to an anti-idiotypic monoclonal antibody which specifically binds to an anti-sialyl-Lewis A monoclonal antibody; and
    (b) detecting the formation of an anti-idiotypic monoclonal antibody/anti-sialyl-Lewis A antibody complex.

10. A method for identifying a patient with cancer comprising the steps of:
    (a) immobilizing an anti-idiotypic monoclonal antibody specifically binding to anti-sialyl-Lewis A monoclonal antibody on a solid phase;
    (b) contacting a sample to said solid phase;
    (c) contacting a labeled anti-idiotypic monoclonal antibody which specifically binds to anti-sialyl-Lewis A monoclonal antibody to said solid phase; and
    (d) detecting the formation of an anti-sialyl-Lewis A antibody/labeled anti-idiotypic monoclonal antibody complex on said solid phase by means of said label.

11. A kit comprising:
    (a) an anti-idiotypic monoclonal antibody specifically binding to anti-sialyl-Lewis A monoclonal antibody on a solid phase; and
    (b) a labeled anti-idiotypic monoclonal antibody specifically binding to anti-sialyl-Lewis A monoclonal antibody.

12. A method for identifying a patient with cancer comprising the steps of:
    (a) immobilizing an anti-idiotypic monoclonal antibody specifically binding to anti-sialyl-Lewis A monoclonal antibody on a solid phase;
    (b) contacting a sample to said solid phase;
    (c) contacting a labeled anti-human immunoglobulin specific antibody to said solid phase; and
    (d) detecting the formation of an anti-sialyl-Lewis A antibody/labeled anti-human immunoglobulin specific antibody complex on said solid phase by means of said label.

13. A kit comprising:
    (a) an anti-idiotypic monoclonal antibody specifically binding to anti-sialyl-Lewis A monoclonal antibody on a solid phase; and
    (b) a labeled anti-human immunoglobulin specific antibody.

14. The use of an anti-idiotypic monoclonal antibody as defined in anyone of claims 1 to 4 for preparing a pharmaceutical composition for the treatment of cancer.

Fig. 1

OD415

Fig. 2

EP 0 508 282 A2

Fig. 3

Sialyl Le$^a$ oligosaccharide (µg/ml)

Fig. 4

(a)　　　　　　　　OD415

Inhibition

(b)

Fig. 5

Fig. 6

Day after tumor transplantation

Fig. 7

OD415